# EUROPEAN PATENT SPECIFICATION

(11) **EP 3 294 259 B1**
(45) Date of publication and mention of the grant of the patent: **01.04.2020**
(21) Application number: 16716619.8
(22) Date of filing: 18.04.2016
(51) Int. Cl.: A61K 8/25, A61Q 11/00, A61K 8/19

(54) **ORAL CARE COMPOSITION**
MUNDPFLEGEZUSAMMENSETZUNG
COMPOSITION DE PROTECTION ORALE

(30) Priority: 13.05.2015 WO PCT/CN2015/078847; 23.06.2015 WO PCT/EP2015/173259
(43) Date of publication of application: 21.03.2018
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); WANG, Jinfang, Shanghai 200335 (CN); ZHANG, Meili, Shanghai 200335 (CN)
(74) Representative: Tansley, Sally Elizabeth
(86) International application number: PCT/EP2016/058495
(87) International publication number: WO 2016/180601

(56) References cited:
- EP-A1- 2 572 698
- WO-A1-02/30381
- WO-A1-2008/068149
- WO-A1-2011/109919
- WO-A2-2012/031785
- WO-A2-2014/056713
- US-A- 5 788 951

## Description

### Technical Field of the Invention

The present invention relates to dual-phase oral care compositions such as tooth pastes, gums and the like. In particular the present invention relates to a dual-phase oral care composition that results in remineralization and/or whitening of teeth by delivering calcium and silicate sources according to claim 1, to the teeth of an individual when, for example, brushing teeth. The invention also relates to the use of such compositions for remineralizing and/or whitening of teeth of an individual.

### Background of the Invention

The enamel layer of the tooth is naturally an opaque white or slightly off-white colour. However, this enamel layer can become stained or discoloured. The enamel layer of the tooth is composed of hydroxyapatite mineral crystals that create a somewhat porous surface. It is believed that this porous nature of the enamel layer allows staining agents and discolouring substances to permeate the enamel and discolour the tooth.

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discolouring substances are often able to permeate the enamel layer. This problem occurs gradually over many years, but imparts a noticeable discoloration of the enamel of one's teeth.

In addition to what we consume, the natural equilibrium between tooth hydroxyapatite being dissolved from the enamel of teeth and hydroxyapatite being formed on or in teeth from substances occurring naturally in the saliva shifts continuously. Such a shift can yield unattractive teeth with cariogenic conditions. A variety of products are currently used for addressing tooth decay and/or tooth whitening. Such products often comprise peroxides, abrasives or both. These types of products are often not desired since they do not contribute to the remineralization of teeth and can cause damage to teeth and gums if overused. Products comprising calcium source have been developed in an attempt to enhance tooth remineralization.

The present inventors have now developed a dual-phase oral care composition which delivers a calcium source to the surface of the teeth and forms calcium silicate hydrate *in situ* by the simultaneous or sequential application of a water soluble silicate source. Such *in situ* formed calcium silicate hydrate adheres to tooth enamel, dentin or both and that is precursor for hydroxyapatite formation. The calcium source and the silicate source are physically separate prior to the use of the oral care composition and are delivered from independent phases to prevent the premature reaction between the two sources. It has been found unexpectedly that such *in situ* formed calcium silicate hydrate behaves much more active than pre-formed calcium silicate hydrate for tooth remineralization. Besides, by using *in situ* formed calcium silicate hydrate instead of pre-formed calcium silicate hydrate, it helps to simplify the manufacturing process and reduce the cost. Additionally, it has been further found that *in situ* formed calcium silicate hydrate can behave as a deposition aid and enhance the deposition of particulate tooth whitening agent on tooth surfaces to deliver tooth whitening benefits.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2012/031785 A (Unilever) discloses composite particle actives suitable for use in oral care compositions. The composite particle actives have a core and a coating whereby the coating interacts with phosphate ions to produce calcium and phosphate reaction products that are suitable to adhere to tooth enamel and/or dentin in order to improve characteristics of teeth.

WO 2012/031786 A (Unilever) discloses oral care compositions with composite particle actives described in WO 2012/031785 A (Unilever).

None of the references above describes a dual-phase oral care composition which comprises a first phase comprising a calcium source and a second phase comprising a water soluble silicate source, particularly the calcium source and the silicate source are delivered to the surface of teeth simultaneously or sequentially to form calcium silicate hydrate *in situ* that adhere to tooth enamel, dentin or both and that is precursor for hydroxyapatite formation.

### Tests and Definitions

### Dual-Phase

"Dual-Phase" for the purposes of the present invention means a composition having two independent phases which are physically separate.

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size", as used herein, refers to particle diameter unless otherwise stated. Diameter is meant to mean the largest measurable distance or a leading dimension on a particle in the event a well-defined sphere is not generated. Particle size can be measured, for example, by dynamic light scattering (DLS).

### Composite Particle

"Composite particle", as used herein, refers to a particle comprising a first component core and a second component coating wherein the core and coating are composed of different materials.

### Refractive Index

Refractive index is quoted at a temperature of 25°C and a wavelength of 589 nm.

### Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of higher than 0.001 moles per litre and less than 0.1 moles per litre.

### Substantially Free

"Substantially free of', as used herein, means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Hydrous Composition

"Hydrous composition", as used herein, means the composition comprises water higher than 1.5%, preferably higher than 5%, more preferably higher than 10% and most preferably from 20 to 90% by weight of the composition.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### In Situ

*"In situ",* as used herein, means during and/or after application of the oral care composition to the oral cavity.

### Remineralization

"Remineralization", as used herein, means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Deposition Aid

Deposition aid, as used herein, means a material which aids deposition of particulate tooth whitening agent from the continuous phase of an oral care composition onto the tooth surface during use of the composition.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to a dual-phase oral care composition comprising:
a) a first phase comprising a calcium source according to claim 1;
b) a second phase comprising a water soluble silicate source according to claim 1; and
wherein the calcium source and the silicate source are physically separate prior to the use of the composition.

In a second aspect, the present invention is directed to a packaged oral care product comprising the oral care composition of the first aspect of this invention.

In a third aspect, the present invention is directed to a method of remineralizing and/or whitening of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that calcium source according to claim 1 and silicate source according to claim 1 delivered to the surfaces of teeth simultaneously or sequentially can form calcium silicate hydrate *in situ* that adhere to tooth enamel, dentin or both and that is precursor for hydroxyapatite formation. It has been found unexpectedly that such *in situ* formed calcium silicate hydrate behaves much more active than pre-formed calcium silicate hydrate for tooth remineralization. Besides, by using *in situ* formed calcium silicate hydrate instead of pre-formed calcium silicate hydrate, it helps to simplify the manufacturing process and reduce the cost. Additionally, it has been further found that the *in situ* formed calcium silicate hydrate can behave as a deposition aid and enhance the deposition of particulate tooth whitening agent on tooth surfaces to deliver tooth whitening benefits.

The calcium source is calcium hydroxide, calcium gluconate, calcium oxide, calcium lactate, calcium sulfate, calcium carboxymethyl cellulose, calcium alginate, calcium salts of citric acid, calcium chloride, calcium nitrate, calcium acetate, mixtures thereof or the like. In a preferred embodiment, the calcium source is calcium hydroxide, calcium nitrate, calcium sulfate or mixtures thereof.

In an especially preferred embodiment, the calcium source according to claim 1 is water soluble and/or slightly soluble.

Typically, the content of the calcium source according to claim 1 is from 0.1 to 50%, more preferably from 0.2 to 30%, most preferably from 1 to 20%, based on the total weight of the oral care composition and including all ranges subsumed therein.

Preferably, the first phase of the oral care composition is substantially free of silicate ions. The silicate source according to claim 1 suitable for use in this invention is limited only to the extent that the same may be used in the mouth. The silicate as used herein is oxide, which contains a silicon oxoanions. Preferably, the silicate comprises or is orthosilicate having the anion SiO₄⁴⁻.

In a preferred embodiment, the silicate source is water soluble. The types of silicate source suitable for use in this invention are sodium silicate, potassium silicate, tetraethyl orthosilicate, tetraethylsilicate, mixtures thereof. In a preferred embodiment, the silicate source is sodium silicate.

Typically, the content of the silicate source according to claim 1 is from 0.5 to 50%, more preferably from 2 to 40%, and most preferably from 5 to 35%, based on the total weight of oral care composition and including all ranges subsumed therein.

Preferably, the second phase of the oral care composition is substantially free of calcium ions.

The oral care composition preferably comprises the calcium source and silicate source according to claim 1, in a molar ratio (Ca:Si) less than 1:1, more preferably less than 1:1.2, more preferably still from 1:1.5 to 1:4 and most preferably from 1:1.7 to 1:3.

The oral care composition of this invention may further comprise particulate whitening agents such as high refractive index particles. The only limitation with respect to the high refractive index particles that may be used in this invention is that the same is suitable for use in the mouth.

It is believed that the deposition of high refractive index particles onto the tooth surfaces is aided by the dual-phase composition. The delivery of calcium and silicate ions from independent phases forms calcium silicate hydrate *in situ* which results in hydroxyapatite formation that aids the deposition of high refractive particles.

In order to provide excellent whitening effect, the particles of the present invention are preferred to have a high refractive index of at least 1.9, more preferably at least 2.0, even more preferably at least 2.2, even more preferably still at least 2.4 and most preferably at least 2.5. The maximum refractive index of the high refractive index particles is not particularly limited but preferably up to 4.0.

Typically, the high refractive index particle comprises a material suitable to physically and immediately improve characteristics of teeth and especially whiten teeth. Particularly suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the high refractive index particle can also comprise non-metal oxides such as silicon monoxide (SiO).

In a preferred embodiment, the high refractive index particle comprises metal oxides, non-metal oxides or a combination thereof in an amount of at least 50% by weight of the particle and more preferably at least 70%, more preferably still from 80 to 100% and most preferably from 85 to 95%. In an especially preferred embodiment, the high refractive index particle is at least 50% by weight titanium dioxide, and most preferably, from 60 to 100% by weight titanium dioxide, based on total weight of the high refractive index particle and including all ranges subsumed therein. In another especially preferred embodiment, the high refractive index particles are slightly soluble or insoluble in water, but most preferably, insoluble in water.

In a preferred embodiment, the high refractive index particles are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. Particular suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all above-mentioned ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium slats of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the high refractive index particle is titanium dioxide coated with calcium silicate.

The high refractive index particles according to the present invention can be of different sizes and shapes. The particles may be of spherical, platelet or irregular shape form. The diameter of the high refractive index particles is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

Typically, the oral care composition of the present invention comprises from 0.25 to 60%, and preferably, from 0.5 to 40%, and most preferably, from 1 to 30% by weight of the high refractive index particles, based on the total weight of the oral care composition and including all ranges subsumed therein.

The oral care composition of the present invention is found to be capable of remineralization of teeth *in situ* without inclusion of a phosphate source in the composition itself. Without wishing to be bound by the theory the present inventors believe that this may be because the Si-OH groups may have an affinity for Ca ions in teeth, and the *in situ* formed calcium silicate hydrate reacts with phosphate ions in saliva that leads to hydroxyapatite formation.

Thus in one embodiment the composition may be substantially free of phosphate source.

Preferably, the dual-phase oral care composition comprises a phosphate source.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of remineralizing and/or whitening of teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for remineralization of the teeth of an individual. Alternatively and preferably, the use is non-therapeutic.

The calcium and the silicate sources according to claim 1, are kept physically separate from one another by having them in independent phases. The delivery of the two independent phases to the teeth may be simultaneous or sequential. In a preferred embodiment, the phases are delivered simultaneously. Typically, the dual-phase composition is delivered by a dual-tube having a first compartment for the first phase comprising calcium source according to claim 1 and a second compartment for the second phase comprising the silicate according to claim 1 which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the phase as the core comprises the calcium source and the sheath phase comprises the silicate source.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first comprising the calcium source according to claim 1 and the second comprising the silicate source. according to claim 1. The means of delivery may involve a cotton rod, or a tray, onto which the calcium source and the silicate source are applied, prior to the tray being placed in contact with the teeth.

The dual-phase oral care composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 30 s to 5 minutes. The composition may be used daily, for example for use by an individual once, twice or three times per day. In another embodiment, when the dual-phase composition is a gel composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

### Examples

### Example 1

This example demonstrates the improved deposition on tooth surface of *in situ* formed calcium silicate hydrate (CSH) compared to pre-formed CSH. All ingredients are expressed by weight percent of each phase of the total formulation, and as level of active ingredient.

**TABLE 1**

| Ingredient | Sample 1 | | Sample 2 | | Sample 3 | | Sample 4 | | Sample 5 |
|---|---|---|---|---|---|---|---|---|---|
| | Calcium Phase | Silicate Phase | Calcium Phase | Silicate Phase | Calcium Phase | Silicate Phase | Calcium Phase | Silicate Phase | |
| Pre-formed CSH | - | - | - | - | -- | -- | -- | -- | 9.1 |
| Na₂SiO₃ | - | 19.6 | - | 19.6 | -- | 19.6 | -- | 19.6 | - |
| Ca(OH)₂ | 6.9 | - | - | - | -- | -- | -- | -- | - |
| CaSO₄·0.5H₂O | - | - | - | - | 12.7 | -- | -- | -- | - |
| Ca(NO₃)₂·4H₂O | - | - | - | - | - | -- | 19.1 | -- | - |
| CaCl₂ | - | - | 10.1 | - | -- | -- | -- | -- | - |
| Water | Balance | Balance | Balance | Balance | Balance | Balance | Balance | Balance | Balance |

**TABLE 2**

| Ingredient | Sample 6 | | Sample 7 | | Sample 8 | | Sample 9 | |
|---|---|---|---|---|---|---|---|---|
| | Calcium Phase | Silicate Phase | Calcium Phase | Silicate Phase | Calcium Phase | Silicate Phase | Calcium Phase | Silicate Phase |
| Na₂SiO₃ | -- | 32.8 | -- | 32.8 | - | 32.8 | - | 32.8 |
| Ca(OH)₂ | 12.9 | -- | -- | -- | - | - | - | - |
| CaSO₄·0.5H₂O | -- | -- | -- | -- | 22.6 | - | - | - |
| Ca(NO₃)₂·4H₂O | -- | -- | -- | -- | - | - | 32.1 | - |
| CaCl₂ | -- | -- | 18.3 | -- | - | - | - | - |
| Glycerin | Balance | Balance | Balance | Balance | Balance | Balance | Balance | Balance |

### Preparation of calcium silicate hydrate powder

The pre-formed CSH was prepared using a mixture of calcium hydroxide and sodium silicate in deionised water. The mixture was formed with an initial Ca:Si ratio of 1:2. The mixture was continuously stirred at room temperature (25°C). The pH of the reaction mixture was adjusted to and maintained around 11 using hydrochloric acid. After stirring for 5 hours the reaction mixture was filtered and the cake washed three times with water before again being filtered. The filter cake was dried in an oven at 80°C for 12 hours to yield the final CSH powder.

### Methods

For Samples 1 to 4 and Samples 6 to 9, the calcium phase and silicate phase were prepared in a molar ratio of 1:2 which is the same Ca/Si molar ratio as for the pre-formed CSH.

To evaluate deposition on enamel surface, for Samples 1 to 4, the silicate phase was first added onto enamel surface, then the brushing was started when the calcium phase was also added. For Samples 6 to 9, the silicate phase and the calcium phase were first mixed and added onto enamel surface, then the brushing was started when 10 mL water was added. For Sample 5, a slurry was prepared by mixing 1 g of pre-formed CSH with 10 mL water and poured onto the enamel surface.

The bovine enamel blocks were treated with different samples via brushing following the same protocol. The enamel blocks were brushed under a tooth brushing machine equipped with toothbrushes. The load of the tooth brushing was 170 g +/-5 g and the automatic brushing operated at a speed of 150 rpm. After brushing for 3 minutes, the enamel blocks were rinsed with distilled water twice and soaked in simulated oral fluid (SOF) under the condition of a shaking water bath at 37°C and 60.0 rpm. After soaking for about 5 hours, the enamel blocks were brushed with the slurry by machine using the same procedure as in the first step. These steps are considered as a whole treatment cycle. The enamel blocks were treated for 1 and 3 times.

Simulated oral fluid was made by combining the ingredients in Table 3:

**TABLE 3**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.622 |
| 1M HCl | 40ml |
| CaCl₂ | 0.1998 |
| Na₂SO₄ | 0.1434 |
| Buffer | Adjust pH to 7.0 |
| Water | Balance to 2L |

### Results

After one treatment and three treatments, Scanning Electron Microscopy (SEM) images of the enamel block surfaces were taken. From the SEM images, it was apparent that Samples 1 to 4 and Samples 6 to 9 gave more uniform and denser deposition on tooth surface than Sample 5 comprising pre-formed CSH.

Analysis using EDX (Energy Dispersive X-ray Spectroscopy) indentified the elements of Si, Ca and P within the new layer, indicating that calcium silicate (CS) deposited on tooth surface and induced remineralization.

### Example 2

This example demonstrates the improved deposition of particles by using *in situ* formed CSH. All ingredients are expressed by weight percent of each phase of the total formulation, and as level of active ingredient.

**TABLE 4**

| Ingredient | Sample 10 | | Sample 11 | | Sample 12 |
|---|---|---|---|---|---|
| | Calcium Phase | Silicate Phase | Calcium Phase | Silicate Phase | |
| Glycerine | Balance | Balance | Balance | Balance | Balance |
| Pre-formed CSH | -- | - | - | - | 8.3 |
| Na₂SiO₃ | -- | 32.8 | - | 32.8 | - |
| Ca(OH)₂ | 11.0 | - | - | - | - |
| CaCl₂ | -- | - | 15.7 | - | - |
| Calcium silicate coated titanium dioxide^{a} | 14.8 | - | 14.0 | - | 8.3 |

| | | | | | |
|---|---|---|---|---|---|
| a. Commercially available calcium silicate coated titanium dioxide from KOBO Products Inc | | | | | |

### Methods

For Samples 10 and 11, the calcium phase and the silicate phase were first mixed and added onto enamel surface, then the brushing was started when water was added.

For Sample 12, fresh slurry was prepared by mixing the sample with water at a ratio of 5 g to 10 mL water, and used immediately.

The bovine enamel blocks were treated with different samples via brushing following the same protocol. The enamel blocks were brushed under a tooth brushing machine equipped with toothbrushes. The load of the tooth brushing was 170 g +/-5 g and the automatic brushing operated at a speed of 150 rpm. After brushing for 3 minutes, the enamel blocks were rinsed with distilled water twice and soaked in SOF for more than 5 hours under the condition of a shaking water bath at 37°C and 60.0 rpm. After soaking for about 5 hours, the enamel blocks were brushed with the slurry by machine using the same procedure as in the first step. These steps are considered as a whole treatment cycle. The enamel blocks were treated for 5 times. The enamel blocks were further brushed with water before taking the SEM images.

### Results

After one and five treatments, SEM images of the enamel block surfaces were taken. From the SEM images, it showed the formation of a new layer and particles or particles aggregates were uniformly embedded in the newly formed layer for both Samples 10 and 11.

But for Sample 12, the SEM images only showed that particles were randomly attached onto enamel surface. Additionally, for Sample 12, most attached particles were easily brushed away. But for Samples 10 and 11, it clearly showed that the particles remained in the newly formed layer, which indicated the *in situ* formed CSH helps the deposition of particles onto enamel surfaces. Analysis using EDX further indentified the elements of Ti, Ca and P within the new layer, indicating the deposition of particles on tooth surface.

### Example 3

This example shows a dual phase oral care compositions consistent with this invention.

**TABLE 5**

| Ingredients | Percent by weight | |
|---|---|---|
| | Calcium Phase | Silicate Phase |
| Glycerin | 79.24 | 73.24 |
| Sodium monofluorophosphate | 1.11 | 1.11 |
| Sodium saccharin | 0.25 | 0.25 |
| Sodium silicate | -- | 20.00 |
| Xanthan gum | 0.20 | 0.20 |
| Calcium hydroxide | 6.00 | - |
| Silica abrasive | 12.00 | - |
| Sodium lauryl sulfate | -- | 4.00 |
| Flavor | 1.20 | 1.20 |

## Claims

1. A dual-phase oral care composition comprising:
a) a first phase comprising a calcium source;
b) a second phase comprising a silicate source that dissolves in water to give a solution with a concentration of at least 0.1 moles per litre at 25°C and atmospheric pressure; and
wherein the calcium source and the silicate source are physically separate prior to the use of the composition;
wherein the calcium source is calcium hydroxide, calcium gluconate, calcium oxide, calcium lactate, calcium sulfate, calcium carboxymethyl cellulose, calcium alginate, calcium salts of citric acid, calcium chloride, calcium nitrate, calcium acetate or mixtures thereof; and
wherein the silicate source comprises sodium silicate, potassium silicate, tetraethyl orthosilicate, tetraethylsilicate or mixtures thereof.

2. The oral care composition according to claim 1, wherein the calcium source reacts with the soluble silicate source to produce a calcium silicate hydrate *in situ* that adheres to tooth enamel, dentin or both and that is a precursor for hydroxyapatite formation.

3. The oral care composition according to claim 1 or claim 2, wherein the calcium source is calcium hydroxide, calcium nitrate, calcium sulfate or mixtures thereof.

4. The oral care composition according to any of the preceding claims, wherein the calcium source is present in an amount ranging from 1 to 20% by weight of the first phase.

5. The oral care composition according to any of the preceding claims, wherein the silicate source is sodium silicate.

6. The oral care composition according to any of the preceding claims, wherein the molar ratio of calcium source to silicate source is from 1: 1.5 to 1:4, preferably 1:1.7 to 1:3.

7. The oral care composition according to any of the preceding claims, wherein the composition further comprises particles having a refractive index ranging from 1.9 to 4.0, preferably ranging from 2.5 to 4.0.

8. The oral care composition according to claim 7, wherein the high refractive index particles are zinc oxide, titanium dioxide, zirconium dioxide or a mixture thereof, preferably titanium dioxide.

9. The oral care composition according to claim 7, wherein the high refractive index particles are composite particles comprising a first component core comprising a metal compound, preferably the metal is selected from zinc, titanium, zirconium or a mixture thereof, and a second component coating comprising the element calcium, and optionally, potassium, sodium, magnesium, aluminium or a mixture thereof.

10. The oral care composition according to claim 9, wherein the high refractive index particle is titanium dioxide coated with calcium silicate.

11. The oral care composition according to any of the preceding claims, wherein the composition further comprises a phosphate source comprising trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate or mixtures thereof.

12. An oral care composition according to any one of claims 1 to 11 for remineralizing and/or whitening the teeth of an individual comprising the step of applying the composition to at least one surface of the teeth of the individual.

13. The oral care composition for remineralizing and/or whitening the teeth of an individual according to claim 12, wherein the calcium source and the water soluble silicate source are applied from spatially separated phases simultaneously or sequentially, preferably simultaneously.

## Patentansprüche

1. Zweiphasen-Mundpflegezusammensetzung, umfassend:
a) eine erste Phase, umfassend eine Calciumquelle;
b) eine zweite Phase, umfassend eine Silicatquelle, die sich in Wasser löst, um eine Lösung mit einer Konzentration von mindestens 0,1 mol pro Liter bei 25°C und Atmosphärendruck zu liefern; und
wobei die Calciumquelle und die Silicatquelle vor der Verwendung der Zusammensetzung physikalisch getrennt sind;
wobei die Calciumquelle Calciumhydroxid, Calciumgluconat, Calciumoxid, Calciumlaktat, Calciumsulfat, Calciumcarboxymethylcellulose, Calciumalginat, Calciumsalze von Citronensäure, Calciumchlorid, Calciumnitrat, Calciumacetat oder Mischungen davon darstellt; und
wobei die Silicatquelle Natriumsilicat, Kaliumsilicat, Tetraethylorthosilicat, Tetraethylsilicat oder Mischungen davon umfasst.

2. Mundpflegezusammensetzung nach Anspruch 1, wobei die Calciumquelle mit der löslichen Silicatquelle reagiert, um *in situ* ein Calciumsilicathydrat zu bilden, das auf dem Zahnschmelz, dem Dentin oder beiden haftet und das ein Vorläufer für die Hydroxyapatit-Bildung ist.

3. Mundpflegezusammensetzung nach Anspruch 1 oder Anspruch 2, wobei die Calciumquelle Calciumhydroxid, Calciumnitrat, Calciumsulfat oder Mischungen davon ist.

4. Mundpflegezusammensetzung nach irgendeinem der vorhergehenden Ansprüche, wobei die Calciumquelle in einer Menge in dem Bereich von 1 bis 20%, bezogen auf das Gewicht der ersten Phase, vorliegt.

5. Mundpflegezusammensetzung nach irgendeinem der vorhergehenden Ansprüche, wobei die Silicatquelle Natriumsilicat ist.

6. Mundpflegezusammensetzung nach irgendeinem der vorhergehenden Ansprüche, wobei das Molverhältnis der Calciumquelle zur Silicatquelle von 1:1,5 bis 1:4, vorzugsweise von 1:1,7 bis 1:3, beträgt.

7. Mundpflegezusammensetzung nach irgendeinem der vorhergehenden Ansprüche, wobei die Zusammensetzung ferner Partikel umfasst, die einen Brechungsindex in dem Bereich von 1,9 bis 4,0, vorzugsweise in dem Bereich von 2,5 bis 4,0, umfassen.

8. Mundpflegezusammensetzung nach Anspruch 7, wobei die Partikel mit hohem Brechungsindex Zinkoxid, Titandioxid, Zirconiumdioxid oder eine Mischung davon, vorzugsweise Titandioxid, darstellen.

9. Mundpflegezusammensetzung nach Anspruch 7, wobei die Partikel mit hohem Brechungsindex Verbundpartikel darstellen, umfassend einen Kern als erste Komponente, umfassend eine Metallverbindung, wobei das Metall vorzugsweise unter Zink, Titan, Zirconium oder einer Mischung ausgewählt ist, und eine Beschichtung als zweite Komponente, die das Element Calcium und optional Kalium, Natrium, Magnesium, Aluminium oder eine Mischung davon umfasst.

10. Mundpflegezusammensetzung nach Anspruch 9, wobei die Teilchen mit hohem Brechungsindex mit Calciumsilicat beschichtetes Titandioxid darstellen.

11. Mundpflegezusammensetzung nach irgendeinem der vorhergehenden Ansprüche, wobei die Zusammensetzung ferner eine Phosphatquelle umfasst, umfassend Trinatriumphosphat, Mononatriumdihydrogenphosphat, Dinatriumhydrogenphosphat, Natriumpyrophosphat, Tetranatriumpyrophosphat, Natriumhexametaphosphat, Trikaliumphosphat, Monokaliumdihydrogenphosphat, Dikaliumhydrogenphosphat oder Mischungen davon.

12. Mundpflegezusammensetzung nach irgendeinem der Ansprüche 1 bis 11 zum Remineralisieren und/oder Aufhellen der Zähne eines Individuums, umfassend den Schritt des Auftragens der Zusammensetzung auf mindestens eine Oberfläche der Zähne des Individuums.

13. Mundpflegezusammensetzung zum Remineralisieren und/oder Aufhellen der Zähne eines Individuums nach Anspruch 12, wobei die Calciumquelle und die wasserlösliche Silicatquelle aus räumlich getrennten Phasen gleichzeitig oder nacheinander, vorzugsweise gleichzeitig, aufgetragen werden.

## Revendications

1. Composition de soin oral à phase double comprenant :
a) une première phase comprenant une source de calcium ;
b) une seconde phase comprenant une source de silicate qui se dissout dans l'eau pour fournir une solution avec une concentration d'au moins 0,1 mole par litre à 25°C et pression atmosphérique ; et
dans laquelle la source de calcium et la source de silicate se séparent physiquement avant l'utilisation de la composition ;
dans laquelle la source de calcium est l'hydroxyde de calcium, gluconate de calcium, oxyde de calcium, lactate de calcium, sulfate de calcium, calcium carboxyméthylcellulose, alginate de calcium, sels de calcium d'acide citrique, chlorure de calcium, nitrate de calcium, acétate de calcium ou des mélanges de ceux-ci ; et
dans laquelle la source de silicate comprend du silicate de sodium, silicate de potassium, orthosilicate de tétraéthyle, silicate de tétraéthyle ou des mélanges de ceux-ci.

2. Composition de soin oral selon la revendication 1, dans laquelle la source de calcium réagit avec la source de silicate soluble pour produire un hydrate de silicate de calcium *in situ* qui adhère à l'émail de dent, la dentine ou les deux et qui est un précurseur pour la formation d'hydroxyapatite.

3. Composition de soin oral selon la revendication 1 ou la revendication 2, dans laquelle la source de calcium est l'hydroxyde de calcium, nitrate de calcium, sulfate de calcium ou des mélanges de ceux-ci.

4. Composition de soin oral selon l'une quelconque des revendications précédentes, dans laquelle la source de calcium est présente dans une quantité de 1 à 20 % en masse de la première phase.

5. Composition de soin oral selon l'une quelconque des revendications précédentes, dans laquelle la source de silicate est le silicate de sodium.

6. Composition de soin oral selon l'une quelconque des revendications précédentes, dans laquelle le ratio molaire de source de calcium à source de silicate est de 1:1,5 à 1:4, de préférence de 1:1,7 à 1:3.

7. Composition de soin oral selon l'une quelconque des revendications précédentes, dans laquelle la composition comprend de plus des particules ayant un indice de réfraction de 1,9 à 4,0, de préférence de 2,5 à 4,0.

8. Composition de soin oral selon la revendication 7, dans laquelle les particules d'indice de réfraction élevé sont l'oxyde de zinc, dioxyde de titane, dioxyde de zirconium ou un mélange de ceux-ci, de préférence dioxyde de titane.

9. Composition de soin oral selon la revendication 7, dans laquelle les particules d'indice de réfraction élevé sont des particules composites comprenant un noyau de premier constituant comprenant un composé de métal, le métal est de préférence choisi parmi le zinc, titane, zirconium ou un mélange de ceux-ci, et un revêtement de second constituant comprenant le calcium élément, et éventuellement, du potassium, sodium, magnésium, aluminium ou un mélange de ceux-ci.

10. Composition de soin oral selon la revendication 9, dans laquelle la particule d'indice de réfraction élevé est du dioxyde de titane revêtu de silicate de calcium.

11. Composition de soin oral selon l'une quelconque des revendications précédentes, dans laquelle la composition comprend de plus une source de phosphate comprenant du phosphate de trisodium, dihydrogénophosphate de monosodium, hydrogénophosphate de disodium, pyrophosphate de sodium, pyrophosphate de tétrasodium, hexamétaphosphate de sodium, phosphate de tripotassium, dihydrogénophosphate de monopotassium, hydrogénophosphate de dipotassium ou des mélanges de ceux-ci.

12. Composition de soin oral selon l'une quelconque des revendications 1 à 11 pour la reminéralisation et/ou le blanchiment des dents d'un individu comprenant l'étape d'application de la composition sur au moins une surface des dents de l'individu.

13. Composition de soin oral pour la reminéralisation et/ou le blanchiment des dents d'un individu selon la revendication 12, dans laquelle la source de calcium et la source de silicate soluble dans l'eau sont appliquées à partir de phases spatialement séparées simultanément ou successivement, de préférence simultanément.
